# EUROPEAN PATENT APPLICATION

(11) **EP 1 878 714 A1**
(43) Date of publication of application: **16.01.2008**
(21) Application number: 06732016.8
(22) Date of filing: 18.04.2006
(51) Int. Cl.: C07C 5/10, C01B 3/26, C07C 13/18, C07C 13/50, C07B 61/00

(54) **ORGANIC HYDRIDE SYNTHESIZING APPARATUS, ORGANIC HYDRIDE SYNTHESIZING SYSTEM AND HYDROGEN PRODUCTION APPARATUS**

(30) Priority: 02.05.2005 JP 2005133815
(71) Applicant: Hrein Energy, Inc., Hokkaido, 0600002 (JP)
(72) Inventor: SUGAI, Yasunori, c/o HREIN ENERGY, INC., Chuo-ku,S apporo-shi Hokkaido 0600002 (JP); TANABE, Katsumori, c/o DENSEI, INC., Sapporo-shi Hookkaido 0040015 (JP); SAKURAMOTO, Tadashi, c/o DENSEI, INC., Sapporo-shi Hokkaido 0040015 (JP)
(74) Representative: Strobel, Wolfgang
(86) International application number: PCT/JP2006/308087
(87) International publication number: WO 2006/120841

(57) **Abstract**

It is intended to realize storage and transportation of large amounts of energy and to carry out storage or supply of energy in conformity with fluctuation of natural energy. There is provided an organic hydride synthesizing apparatus capable of hydrogenation reaction between an unsaturated hydrocarbon and hydrogen in the presence of a catalyst to thereby accomplish synthesis of an organic hydride, which organic hydride synthesizing apparatus comprises a catalyst, heating means (3a) for heating the catalyst, hydrogen supply rate detection means (25) for detecting the rate of hydrogen supply to the interior of the apparatus, and control means (31),(32),(33) for in accordance with the supply rate, controlling the rate of unsaturated hydrocarbon fed, the rate of product recycled and the temperature of the catalyst.

## Description

### Technical Field

The present invention relates to an organic hydride synthesizing apparatus which synthesizes an organic hydride through a hydrogenation reaction between unsaturated hydrocarbon and hydrogen, an organic hydride synthesizing system which includes a power generation apparatus for utilizing natural energy to generate electricity, an electrolysis apparatus for electrolyzing water produce the hydrogen, and an organic hydride synthesizing apparatus for synthesizing the organic hydride through the hydrogenation reaction, and a hydrogen production apparatus which produces the hydrogen through a dehydrogenation reaction of the organic hydride.

### Background Art

Generally, power generation by natural energy is easy to be affected by the weather. For example, the production of the electricity depends on strong and weak of the wind in the case of wind power generation, and the production of the electricity depends on strong and weak of sunshine and on long and short sunshine hours in the case of solar power generation. Therefore, an electric power storage apparatus such as a battery is required to stably utilize the electric power obtained by the natural energy (for example, see Japanese Patent Application Laid-Open No. 2004-176696).

However, there is a problem described below in the conventional technique. That is, in storing a large amount of energy or in transmitting the energy to a remote place, the energy storage in which the battery is used has a limitation.

In view of the foregoing, an object of the present invention is to store or supply the energy according to a fluctuation in natural energy while a large amount of energy can be stored and transported.

### Disclosure of the Invention

In accordance with a first aspect of the present invention, in order to achieve the above object, there is provided an organic hydride synthesizing apparatus which synthesizes an organic hydride through a hydrogenation reaction between an unsaturated hydrocarbon and hydrogen in the presence of a catalyst, the organic hydride synthesizing apparatus including the catalyst; heating means for heating the catalyst; hydrogen supply rate detection means for detecting a rate of hydrogen supplied to an inside of the apparatus; and control means for controlling an unsaturated hydrocarbon supply rate, a product recycle amount, or a catalyst temperature according to the hydrogen supply rate.

In accordance with a second aspect of the present invention, there is provided an organic hydride synthesizing system including a power generation apparatus which utilizes natural energy to generate electricity, an electrolysis apparatus which is connected to the power generation apparatus, the electrolysis apparatus electrolyzing water to produce hydrogen by utilizing the electricity generated by the power generation apparatus, and an organic hydride synthesizing apparatus which is connected to the hydrogen production apparatus, the organic hydride synthesizing apparatus synthesizing an organic hydride through a hydrogenation reaction between an unsaturated hydrocarbon and the hydrogen in the presence of a catalyst, the organic hydride synthesizing system includes the catalyst; heating means for heating the catalyst; hydrogen supply rate detection means for detecting a rate of hydrogen supplied to an inside of the apparatus; and control means for controlling an unsaturated hydrocarbon supply rate, a product recycle amount, or a catalyst temperature according to the hydrogen supply rate.

When the apparatus or system of the invention is introduced, even if the electric power derived from the natural energy fluctuates, the unsaturated hydrocarbon supply rate, the product recycle amount, or the catalyst temperature is controlled in synchronization with the fluctuation, whereby the effective catalyst reaction can be performed to enhance the organic hydride synthesizing efficiency. Because the organic hydride is an easily-transported substance in which the hydrogen is stored, the organic hydride can be transported using an oil tanker or the like, even if the organic hydride synthesizing apparatus is distant from a hydrogen supply source. Accordingly, the apparatus or system of the invention has an economic advantage because a long pipe line or an electric cable is not required. As used herein, the product recycle amount shall mean an amount of product including unsaturated hydrocarbon recycled from the hydrogenproductionapparatus, the organic hydride, and the like.

In the organic hydride synthesizing system according to the second aspect of the present invention, preferably a battery is provided between the power generation apparatus and the electrolysis apparatus. The electricity is stored according to the fluctuation in natural energy, so that the electricity supplied to the electrolysis apparatus can stably be supplied. Accordingly, the rate of hydrogen supplied to the organic hydride synthesizing apparatus can also be controlled.

In the organic hydride synthesizing system according to the second aspect of the present invention, preferably a buffer tank in which the hydrogen can be stored is provided between the electrolysis apparatus and the organic hydride synthesizing apparatus. Therefore, the fluctuation in hydrogen obtained by the electrolysis apparatus can be reduced to control the rate of hydrogen supplied to the organic hydride synthesizing apparatus.

In accordance with a third aspect of the present invention, there is provided an hydrogen production apparatus which produces hydrogen through a dehydrogenation reaction of an organic hydride in the presence of a catalyst, the hydrogen production apparatus includes the catalyst; heating means for heating the catalyst; hydrogen rate information obtaining means for obtaining information on a necessary hydrogen rate; and control means for controlling a rate of organic hydride supplied to an inside of the apparatus or a catalyst temperature according to the information on the necessary hydrogen rate.

The effective catalyst reaction can be performed to enhance the hydrogen production efficiency by controlling the organic hydride supply rate or catalyst temperature according to the necessary hydrogen rate. Because the organic hydride is an easily-transported substance in which the hydrogen is stored, the organic hydride can be transported using an oil tanker or the like, even if the hydrogen production apparatus is distant from an organic hydride storage site. Accordingly, the apparatus of the invention has an economic advantage because the long pipe line or electric cable is not required.

According to the invention, the energy can be stored or supplied according to the fluctuation in natural energy while a large amount of energy can be stored and transported.

### Brief Description of the Drawings

- Fig. 1: is a view showing configurations of an organic hydride synthesizing system and a hydrogen production apparatus according to an embodiment of the present invention;
- Fig. 2: is a view showing a configuration of a control device (device for controlling a hydrogenation reaction of unsaturated hydrocarbon) shown in Fig. 1;
- Fig. 3: is a view showing a configuration of a control device (device for controlling a dehydrogenation reaction of organic hydride) shown in Fig. 1;
- Fig. 4: is a view partially showing a modification of the organic hydride synthesizing system shown in Fig. 1;
- Fig. 5: is a graph showing result in which catalyst temperature dependence of a conversion rate from toluene into methylcyclohexane is investigated when toluene is used as the unsaturated hydrocarbon; and
- Fig. 6: is a graph showing result inwhich a relationship between a reaction rate constant and a reaction pressure in the hydrogenation reaction from the toluene into the methylcyclohexane is investigated when the toluene is used as the unsaturated hydrocarbon.

### Explanations of Reference Numerals

- 1: wind-power generation apparatus (one mode of power generation apparatus)
- 2: electrolysis apparatus
- 3: organic hydride synthesizing apparatus
- 3a: heater (heating means)
- 5: unsaturated hydrocarbon storage tank
- 7: valve
- 8: control device (including control means)
- 9: organic hydride storage tank
- 13: hydrogen production apparatus
- 13a: heater (heating means)
- 14: organic hydride storage tank
- 16: valve
- 17: control device (including control means)
- 19: unsaturated hydrocarbon storage tank
- 25: sensor (hydrogen supply rate detection means)
- 30: interface
- 31: central processing unit (part of control means)
- 32: valve control unit (part of control means)
- 33: heater control unit (part of control means)
- 34: memory
- 40: interface (part of hydrogen rate information obtaining means)
- 41: central processing unit (part of control means and part of hydrogen rate information obtaining means)
- 42: valve control unit (part of control means)
- 43: heater control unit (part of control means)
- 44: memory
- 50: battery
- 51: buffer tank

### Best Mode for Carrying out the Invention

An organic hydride synthesizing apparatus, an organic hydride synthesizing system, and a hydrogen production apparatus according to embodiments of the present invention will be described in detail with reference to the drawings. Because the organic hydride synthesizing apparatus is included in the organic hydride synthesizing system, the organic hydride synthesizing apparatus of the embodiment is described in the organic hydride synthesizing system of the embodiment.

First, a hydrogenation reaction and a dehydrogenation reaction, utilized in the invention, will briefly be described.

The following three kinds of reaction formulas show the hydrogenation reaction of an unsaturated hydrocarbon. As shown in the reaction formulas, saturated hydrocarbon is produced by the hydrogenation of the unsaturated hydrocarbon.
C₁₀H₈ + 5H₂ → C₁₀H₁₈ (hydrogenation reaction of naphthalene)
C₆H₆ + 3H₂ → C₆H₁₂ (hydrogenation reaction of benzene)
C₇H₈ + 3H₂ → C₇H₁₄ (hydrogenation reaction of toluene)

The following three kinds of reaction formulas show a dehydrogenation reaction of a saturated hydrocarbon. As shown in the reaction formulas, unsaturated hydrocarbon is produced by the dehydrogenation of the saturated hydrocarbon.
C₁₀H₁₈ → C₁₀H₈ + 5H₂ (dehydrogenation reaction of decalin)
C₆H₁₂ → C₆H₆ + 3H₂ (dehydrogenation reaction of cyclohexane)
C₇H₁₄ → C₇H₈ + 3H₂ (dehydrogenation reaction of methylcyclohexane)

Thus, the hydrogen from the outside can be stored by utilizing the hydrogenation reaction of a hydrocarbon-system raw material such as the unsaturated hydrocarbon including a double bond or a triple bond in a chemical bond between carbons. On the other hand, the hydrogen can be supplied to the outside by utilizing the dehydrogenation reaction of the hydrocarbon-system raw material such as the saturated hydrocarbon in which the carbons are singly-bonded. Hereinafter, the hydrocarbon-system raw materials such as decalin, cyclohexane, and methylcyclohexane which can release the hydrogen existing therein to the outside is collectively referred to as saturated hydrocarbon or organic hydride, and the hydrocarbon-system raw materials such as naphthalene, benzene, and toluene which can bond the hydrogen from the outside to store the hydrogen therein are collectively referred to as unsaturated hydrocarbon.

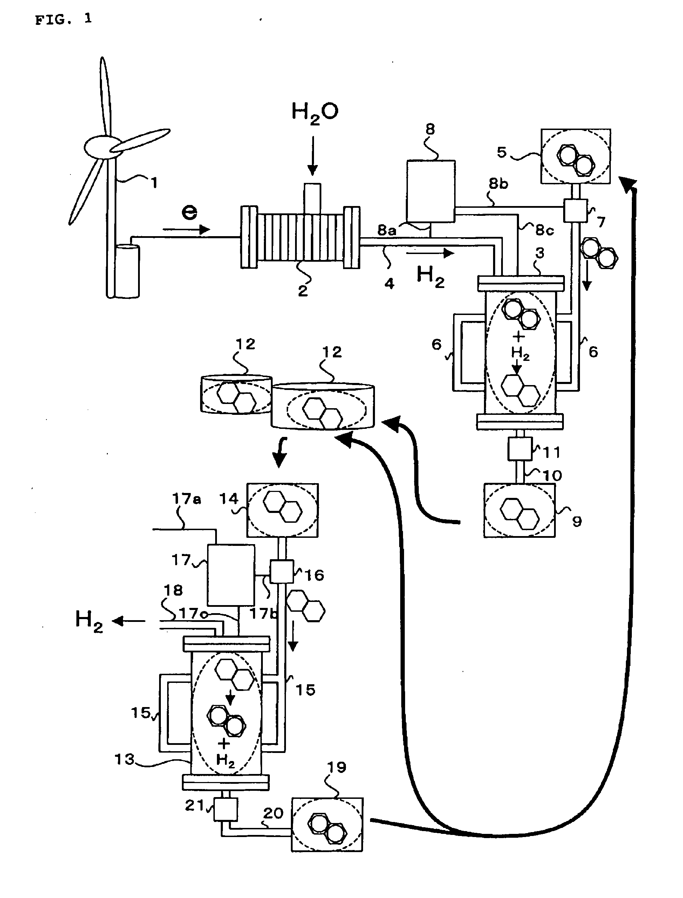

Fig. 1 is a view showing configurations of the organic hydride synthesizing system and hydrogen production apparatus according to the embodiments of the invention. Compounds existing inside and main reactions generated inside are indicated in regions surrounded by oval dotted lines in Fig. 1. Fig. 1 shows an example of the reaction between the naphthalene and the decalin as a typical representative of many reactions.

The organic hydride synthesizing system of the invention mainly includes a wind-power generation apparatus (one mode of the power generation apparatus) 1, an electrolysis apparatus 2, and an organic hydride synthesizing apparatus 3. The wind-power generation apparatus 1 generates the electric power by utilizing the wind power which is of an example of the natural energy. The electrolysis apparatus 2 electrolyzes water by utilizing electricity from the wind-power generation apparatus 1, thereby producing hydrogen. The organic hydride synthesizing apparatus 3 synthesizes the organic hydride by utilizing the hydrogenation reaction between the hydrogen and the unsaturated hydrocarbon. The wind-power generation apparatus 1 and the electrolysis apparatus 2 are connected with an electric cable. The electrolysis apparatus 2 and the organic hydride synthesizing apparatus 3 are connected with a hydrogen pipe 4 through which hydrogen flows.

The organic hydride synthesizing apparatus 3 includes an unsaturated hydrocarbon storage tank 5 in which the unsaturated hydrocarbon is stored, and the organic hydride synthesizing apparatus 3 and the unsaturated hydrocarbon storage tank 5 are connected with a pipe 6. A valve 7 is provided in a midpoint of the pipe 6 to adjust an unsaturated hydrocarbon supply rate. The pipe 6 is branched into plural pipes and inserted from an outer wall of the organic hydride synthesizing apparatus 3 into the inside.

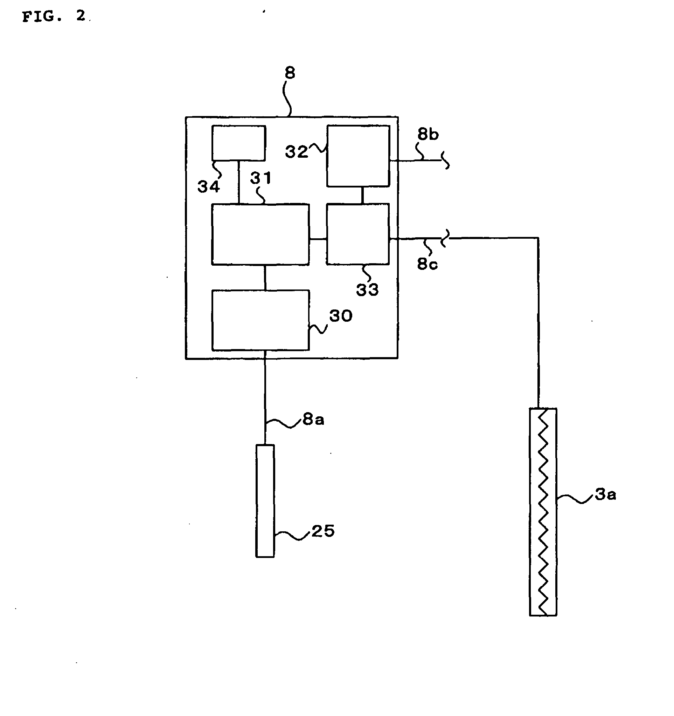

A control device 8 is connected in the midpoint of the hydrogen pipe 4 to control the reaction in the organic hydride synthesizing apparatus 3. Specifically, the control device 8 is connected to the hydrogen pipe 4 in a form that a sensor 25 (described later) connected to a front end of an interconnection 8a extended from the control device 8 is inserted into the hydrogen pipe 4. An electric wiring 8b and an electric wiring 8c which are extended from the control device 8 are electrically connected to a valve 7 and a catalyst heating heater 3a (see Fig. 2) disposed in the organic hydride synthesizing apparatus 3 respectively. Any electromagnetic valve or any air regulator valve accompanied by a compressed air supply device may be used as the valve 7 as long as the valve can receive an electric signal from the control device 8 to control the unsaturated hydrocarbon supply rate.

The organic hydride synthesizing apparatus 3 includes an organic hydride storage tank 9 in which the organic hydride synthesized by the hydrogenation reaction between the unsaturated hydrocarbon and the hydrogen is stored, and the organic hydride synthesizing apparatus 3 is connected to the organic hydride storage tank 9 through a pipe 10. A valve 11 is provided in the midpoint of the pipe 10. For example, the organic hydride stored in the organic hydride storage tank 9 is reserved in a remotely-disposed tank 12. For example, the organic hydride can be transported from the organic hydride storage tank 9 to the tank 12 (path shown by a bold solid line of Fig. 1) using an oil tanker.

Then, a hydrogen production apparatus 13 will be described.

The hydrogen production apparatus 13 includes an organic hydride storage tank 14 in which the organic hydride is stored, and the hydrogen production apparatus 13 is connected to the organic hydride storage tank 14 through a pipe 15. A valve 16 is provided in the mid point of the pipe 15 to adjust the organic hydride supply rate. The pipe 15 is branched into plural pipes and inserted from the outer wall of the hydrogen production apparatus 13 into the inside. In the case of the long path from the tank 12 to the hydrogen production apparatus 13 (path shown by the bold solid line of Fig. 1), for example, the organic hydride can be transported while loaded in the oil tanker.

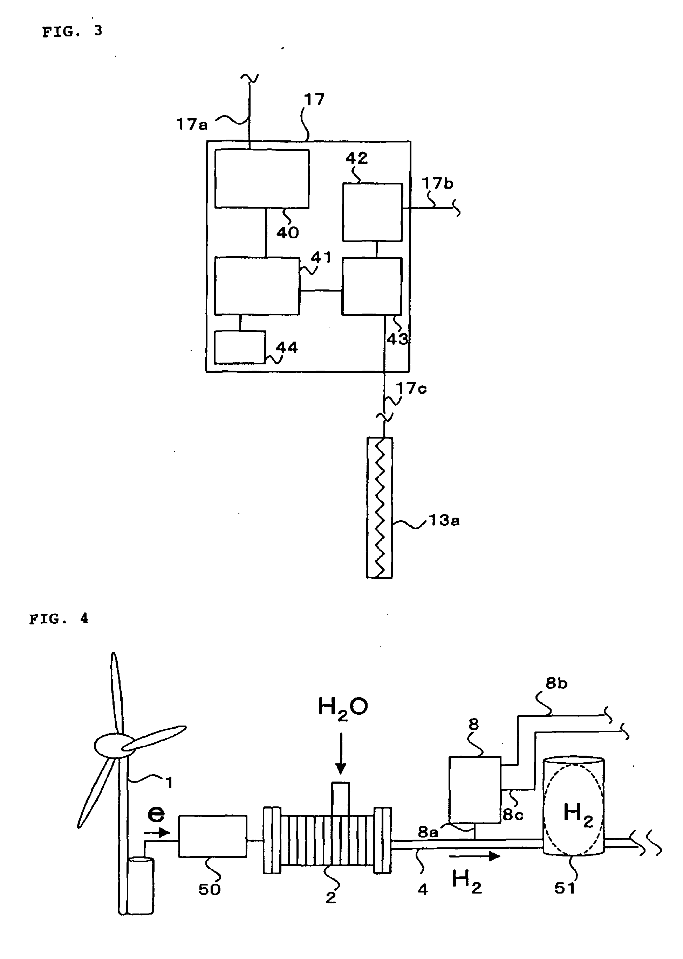

A control device 17 which controls the reaction in the hydrogen production apparatus 13 is connected to the hydrogen production apparatus 13. Specifically, the control device 17 is connected to an external information transmission device (not shown) through a communication line 17a extended from the control device 17. An electric wiring 17b and an electric wiring 17c which are extended from the control device 17 are electrically connected to a valve 16 and a catalyst heating heater 13a (see Fig. 3) disposed in the hydrogen production apparatus 13 respectively. Any electromagnetic valve or any air regulator valve accompanied by a compressed air supply device may be used as the valve 16 as long as the valve can receive the electric signal from the control device 17 to control the organic hydride supply rate.

The hydrogen production apparatus 13 includes a hydrogen supply pipe 18 which supplies the hydrogen produced by the dehydrogenation reaction of the organic hydride to the outside. The hydrogen production apparatus 13 includes an unsaturated hydrocarbon storage tank 19 in which the unsaturated hydrocarbon produced by the dehydrogenation reaction of the organic hydride is stored, and the hydrogen production apparatus 13 is connected to the unsaturated hydrocarbon storage tank 19 through a pipe 20. A valve 21 is provided in the midpoint of the pipe 20. For example, the unsaturated hydrocarbon stored in the unsaturated hydrocarbon storage tank 19 can be reserved in the remotely-disposed unsaturated hydrocarbon storage tank 5 or returned to the tank 12. For example, the unsaturated hydrocarbon can be transported from the unsaturated hydrocarbon storage tank 19 to the unsaturated hydrocarbon storage tank 5 or tank 12 (path shown by the bold solid line of Fig. 1) using the oil tanker.

Fig. 2 is a view showing a configuration of the control device 8.

The control device 8 includes a sensor (one mode of the hydrogen supply rate detection means) 25 which detects a rate of the hydrogen supplied from the electrolysis apparatus 2. The control device 8 includes an interface (I/F) 30 which receives information on the hydrogen supply rate, a central processing unit (CPU) 31 connected to I/F 30 through a bus, a valve control unit 32 which receives a command from CPU 31 to transmit a signal for controlling the valve 7, a heater control unit 33 which receives a command from CPU 31 to transmit a signal for controlling a temperature of the catalyst heating heater 3a in the organic hydride synthesizing apparatus 3, and a memory 34 in which a control program of CPU 31 is stored. CPU 31, the valve control unit 32, and the heater control unit 33 constitute the control means for controlling the unsaturated hydrocarbon supply rate, the product recycle amount, or the catalyst temperature according to the hydrogen supply rate. The product recycle amount shall mean the amount of product including unsaturated hydrocarbon recycled from the hydrogen production apparatus 13, the organic hydride, and the like. The control device 8 may be configured to change the rate of hydrogen produced by the electrolysis apparatus 2 from information on generator output from the wind-power generation apparatus 1 or information on the number of revolutions of the generator.

Any detection method may be adopted in the sensor 25 as long as the hydrogen supply rate can be detected. When the sensor 25 detects the hydrogen supply rate, the information is transmitted to CPU 31 through I/F 30. CPU 31 adjusts an opening of the valve 7 to control the unsaturated hydrocarbon supply rate such that the hydrogenation reaction is performed at a proper conversion rate according to the hydrogen supply rate based on the control program stored in the memory 34. For example, in the case of the high hydrogen supply rate, the opening of the valve 7 is increased to supply a larger amount of unsaturated hydrocarbon into the organic hydride synthesizing apparatus 3.

CPU 31 can also adjust a current passed through the catalyst heating heater 3a such that the hydrogenation reaction is performed at a proper conversion rate according to the hydrogen supply rate based on the control program stored in the memory 34. A user can selectively switch between the adjustment of the current passed through the heater 3a and the adjustment of the opening of the valve 7. Both the adjustment of the current passed through the heater 3a and the adjustment of the opening of the valve 7 may be performed to control the conversion rate of the hydrogenation reaction irrespective of the presence or absence of the user selection.

Fig. 3 is a view showing a configuration of the control device 17.

The control device 17 includes an interface (I/F) 40 which receives information on the necessary hydrogen rate, a central processing unit (CPU) 41 connected to I/F 40 through a bus, a valve control unit 42 which receives a command from CPU 41 to transmit a signal for controlling the valve 16, a heater control unit 43 which receives a command from CPU 41 to transmit a signal for controlling a temperature of the catalyst heating heater 13a in the hydrogen production apparatus 13, and a memory 44 in which a control program of CPU 41 is stored. I/F 40 and CPU 41 constitute the hydrogen rate information obtaining means for obtaining the information on the necessary hydrogen rate. CPU 41, the valve control unit 42, and the heater control unit 43 constitute the control means for controlling the rate of organic hydride supplied into the apparatus or the catalyst temperature based on the necessary hydrogen rate.

The information on the necessary hydrogen rate is transmitted from the outside to CPU 41 through I/F 40. CPU 41 adjusts the opening of the valve 16 to control the organic hydride supply rate such that the dehydrogenation reaction is performed at a proper conversion rate according to the hydrogen supply rate based on the control program stored in the memory 44. For example, in the case of the high hydrogen supply rate, the opening of the valve 16 is increased to supply a larger amount of organic hydride into the hydrogen production apparatus 13.

CPU 41 can also adjust the current passed through the catalyst heating heater 13a such that the dehydrogenation reaction is performed at a proper conversion rate according to the necessary hydrogen rate based on the control program stored in the memory 44. A user can selectively switch between the adjustment of the current passed through the heater 13a and the adjustment of the opening of the valve 16. Both the adj ustment of the current passed through the heater 13a and the adjustment of the opening of the valve 16 may be performed to control the conversion rate of the dehydrogenation reaction irrespective of the presence or absence of the user selection.

Fig. 4 is a view partially showing a modification of the organic hydride synthesizing system of the embodiment described above.

The organic hydride synthesizing system shown in Fig. 4 includes a battery 50 provided between the wind-power generation apparatus 1 and the electrolysis apparatus 2 and a buffer tank 51 provided between the electrolysis apparatus 2 and the organic hydride synthesizing apparatus 3. An electric quantity supplied to the electrolysis apparatus 2 can be controlled to a certain extent by providing the battery 50. The rate of hydrogen supplied to the organic hydride synthesizing apparatus 3 can be controlled to a certain extent by providing the buffer tank 51. At least either one of the battery 50 and the buffer tank 51 can be disposed if needed.

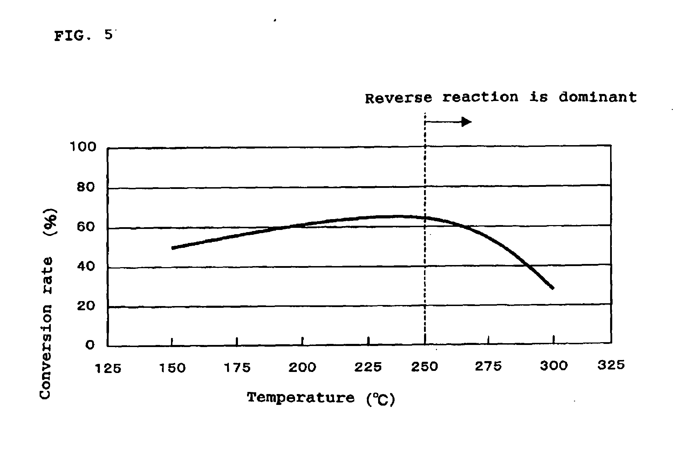

Fig. 5 is a graph showing result in which catalyst temperature dependence of the conversion rate from the toluene into the methylcyclohexane is investigated when the toluene is used as the unsaturated hydrocarbon.

As is clear from Fig. 5, the conversion rate of the toluene into the methylcyclohexane fluctuates when the catalyst temperature is changed. This shows that the hydrogen storage amount can be utilized to control the hydrogen storage amount by adjusting the catalyst temperature. As can be seen the result of Fig. 5, the conversion rate is lowered when the catalyst temperature exceeds about 250°C, while the conversion rate is gradually raised until the catalyst temperature reaches about 250°C. When the catalyst temperature is excessively increased, it is thought that the dehydrogenation reaction in which the methylcyclohexane is converted into the toluene becomes dominant.

In the case where the toluene is used as the unsaturated hydrocarbon, preferably the catalyst temperature in the organic hydride synthesizing apparatus 3 is controlled in a range of 150 to 250°C. Preferably, the catalyst temperature in the hydrogen production apparatus 13 is controlled in the range of 280 to 400°C. When the catalyst temperature exceeds 400°C, undesirably coking of the catalyst is easy to occur.

The conversion rate of the toluene into the methylcyclohexane fluctuates even if the toluene supply rate is changed while the catalyst temperature is kept constant. This shows that the hydrogenation reaction can be utilized to control the hydrogen storage amount by adjusting the toluene supply rate. The same holds true for the dehydrogenation reaction, the hydrogen production rate can be controlled by changing the catalyst temperature or the methylcyclohexane supply rate.

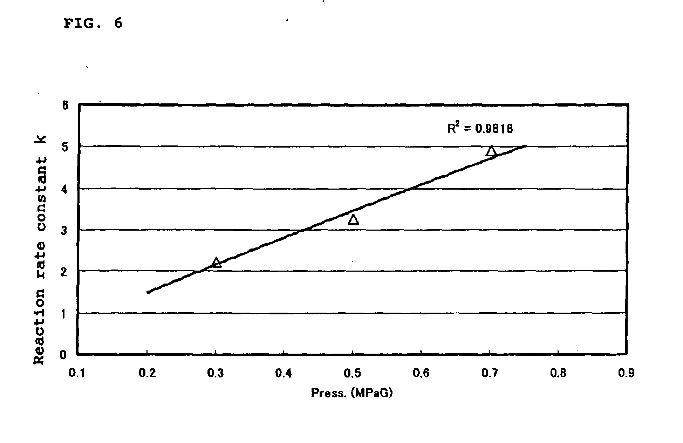

Fig. 6 is a graph showing result in which a relationship between a reaction rate constant and a reaction pressure in the hydrogenation reaction from the toluene into the methylcyclohexane is investigated when the toluene is used as the unsaturated hydrocarbon.

As is clear from Fig. 6, the reaction rate constant of the hydrogenation reaction from the toluene into the methylcyclohexane is increased with increasing reaction pressure. This shows that the hydrogenation reaction can be utilized to control the hydrogen storage amount by adjusting the reaction pressure. When the hydrogen rate becomes excessive, the reaction pressure is increased, whereby the reaction rate constant is also increased. Therefore, the many hydrogens can be added to increase the methylcyclohexane production amount. On the other hand, in the case of the low hydrogen rate, the reaction pressure is decreased, whereby the reaction rate constant is also decreased. Therefore, the methylcyclohexane production amount is decreased. Thus, in the system of the invention, even if the rate of hydrogen produced from the natural energy fluctuates, the fluctuation can sufficientlybe absorbed.

The granular platinum catalyst is used as the catalyst in the embodiment. Alternatively, at least one of powdery, cloth, nonfinite form chip, cylindrical, plate-like, honeycomb, nonfinite form solid-state film platinum catalysts or a combination thereof may be used as the catalyst. The catalyst may be made of one of palladium, ruthenium, iridium, rhenium, nickel, molybdenum, tungsten, nitenium, vanadium, osmium, chromium, cobalt, and iron or an arbitrary combination thereof. The catalyst bearing material may be made of activated carbon, alumina, or metal.

Although the embodiment of the invention is described above, the invention is not limited to the above embodiment, but various modifications can be made in the invention.

For example, in addition to the wind-power generation apparatus 1, pieces of power generation apparatus such as a solar power generation apparatus, a geothermal power generation apparatus, and a hydraulic power generation apparatus in which other pieces of natural energy are utilized may be adopted as the power generation apparatus. A burner may be used as the heating means instead of the heaters 3a and 13a to control a fuel rate from a fuel tank connected to the control devices 8 and 17. In such cases, the control devices 8 and 17 control not the current passed through the heaters 3a and 13a but the rate of fuel supplied to the burner.

The hydrogen production apparatus 13 may obtain not only the information on the necessary hydrogen rate from the outside by the communication, but also the information on the necessary hydrogen rate which is inputted from the control device 17 by a manager. Similarly, the organic hydride synthesizing apparatus 3 does not obtain the information on the hydrogen supply rate through the sensor 25, but the organic hydride synthesizing apparatus 3 may obtain the information on the hydrogen supply rate inputted from the control device 8 by the manager. In the modification shown in Fig. 4, the buffer tank 51 may be provided between the inserted portion of the sensor 25 and the electrolysis apparatus 2.

### Industrial Applicability

The present invention can be applied to industries in which the hydrogen is stored or supplied by utilizing the natural energy.

## Claims

1. An organic hydride synthesizing apparatus which synthesizes an organic hydride through a hydrogenation reaction between an unsaturated hydrocarbon and hydrogen in the presence of a catalyst, the organic hydride synthesizing apparatus **characterized by** comprising:
the catalyst;
heating means for heating the catalyst;
hydrogen supply rate detection means for detecting a rate of hydrogen supplied to an inside of the apparatus; and
control means for controlling an unsaturated hydrocarbon supply rate, a product recycle amount, or a catalyst temperature according to the hydrogen supply rate.

2. An organic hydride synthesizing system including a power generation apparatus which utilizes natural energy to generate electricity, an electrolysis apparatus which is connected to the power generation apparatus, the electrolysis apparatus electrolyzing water to produce hydrogen by utilizing the electricity generated by the power generation apparatus, and an organic hydride synthesizing apparatus which is connected to the electrolysis apparatus, the organic hydride synthesizing apparatus synthesizing an organic hydride through a hydrogenation reaction between an unsaturated hydrocarbon and the hydrogen in the presence of a catalyst, the organic hydride synthesizing system **characterized by** comprising:
the catalyst;
heating means for heating the catalyst;
hydrogen supply rate detection means for detecting a rate of hydrogen supplied to an inside of the organic hydride synthesizing apparatus; and
control means for controlling an unsaturated hydrocarbon supply rate, a product recycle amount, or a catalyst temperature according to the hydrogen supply rate.

3. The organic hydride synthesizing system according to claim 2, **characterized in that** a battery is provided between the power generation apparatus and the electrolysis apparatus.

4. The organic hydride synthesizing system according to claim 2 or 3, **characterized in that** a buffer tank in which the hydrogen canbe stored is providedbetween the electrolysis apparatus and the organic hydride synthesizing apparatus.

5. A hydrogen production apparatus which produces hydrogen through a dehydrogenation reaction of an organic hydride in the presence of a catalyst, the hydrogen production apparatus **characterized by** comprising:
the catalyst;
heating means for heating the catalyst;
hydrogen rate information obtaining means for obtaining information on a necessary hydrogen rate; and
control means for controlling a rate of organic hydride supplied to an inside of the apparatus or a catalyst
temperature according to the information on the necessary hydrogen rate.
